# EUROPEAN PATENT SPECIFICATION

(11) **EP 2 424 977 B1**
(45) Date of publication and mention of the grant of the patent: **12.06.2013**
(21) Application number: 10721372.0
(22) Date of filing: 28.04.2010
(51) Int. Cl.: C12N 5/07, A61P 35/00, A61K 35/30

(54) **MICROGLIAL PRECURSOR CELLS FOR THE TREATMENT OF MALIGNANT NEOPLASMS OF THE CENTRAL NERVOUS SYSTEM**
MIKROGLIALE VORLÄUFERZELLEN ZUR BEHANDLUNG MALIGNER NEOPLASMEN DES ZENTRALEN NERVENSYSTEMS
Cellules de précurseur gliales du cerveau pour le traitement des tumeurs malignes du système nerveux central

(30) Priority: 28.04.2009 EP 09005880
(43) Date of publication of application: 07.03.2012
(73) Proprietor: Life & Brain GmbH, 53127 Bonn (DE); Rheinische Friedrich-Wilhelms-Universität, 53113 Bonn (DE)
(72) Inventor: NEUMANN, Harald, 53123 Bonn (DE); GLAS, Martin, 53115 Bonn (DE); MASGUTOV, Ruslan, Kazan 420049 (RU); HERRLINGER, Ulrich, 53347 Alfter-Impekoven (DE); WELLE, Kristian, 53119 Bonn (DE)
(74) Representative: Castell, Klaus
(86) International application number: PCT/EP2010/055725
(87) International publication number: WO 2010/125107

(56) References cited:
- WO-A-2008/038127
- WO-A-2009/039442
- US-A1- 2003 133 908
- DATABASE MEDLINE [Online] US NATIONAL LIBRARY OF MEDICINE (NLM), BETHESDA, MD, US; 15 February 2009 (2009-02-15), HAN XIAODI ET AL: "Inhibition of intracranial glioma growth by endometrial regenerative cells." XP002548662 Database accession no. NLM19197154 & CELL CYCLE (GEORGETOWN, TEX.) 15 FEB 2009, vol. 8, no. 4, 15 February 2009 (2009-02-15), pages 606-610, ISSN: 1551-4005
- NAPOLI I. ET AL: "Micorglial Precursors Derived from Mouse Embryonic Stem Cells" GLIA, vol. 57, 27 March 2009 (2009-03-27), pages 1660-1671, XP002548661

## Description

The present invention relates to microglial precursor cells for use in the treatment of malignant neoplasms of the central nervous system. The present invention further relates to a method of treating malignant neoplasms of the central nervous system comprising administering a pharmaceutically effective amount of microglial precursor cells to a subject in need thereof.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Gliomas are highly aggressive brain tumors. They are also referred to in the art as malignant gliomas. One major problem of malignant gliomas is their infiltrative growth pattern, with single tumor cells migrating distantly from the solid tumor mass and infiltrating normal brain tissue. Thus, they are difficult to target; whereas elimination of a significant amount of the tumor mass can be accomplished by surgery and subsequent radiotherapy, relapse nonetheless invariably follows within a short amount of time due to tumor cells that have escaped focal therapies. In addition, glial tumor cells inhibit immune responses by secreting strong immunosuppressive mediators. Transforming growth factor-β (TGF-β) is one known factor, which interferes with the immune response by inhibiting the activation of T and NK cells (Weller and Fontana, 1995). Furthermore, glial tumor cells express the anti-inflammatory cytokine interleukin-10 (IL-10) (Nitta, 1998).

Several groups have shown that neural and mesenchymal stem cells (NSCs and MSCs) migrate specifically to and into malignant glioma and even track infiltrating tumor cells. Due to this property, these cells have been suggested as delivery vehicles for therapeutic antitumor agents.

Herrlinger *et al.* (2000) describe the use of neural precursor cells, which can migrate and differentiate in the brain, as a cellular delivery vehicle for herpes simplex virus type 1 vectors for gene therapy of invasive tumor cells in the brains.

Aboody et *al.* (2000) investigate the migratory behaviour of neural stem cells in relation to aggressively invasive tumors in the adult brain. The authors observed a reduction in tumor mass when employing NSCs stably transduced with a transgene encoding cytosine deaminase, which converted the nontoxic prodrug 5-FC to the oncolytic drug 5-fluorouracil, a chemotherapeutic agent.

Uhl *et al.* (2005) also describe gene therapy of glioma based on viral delivery of herpes simplex virus type I thymidine kinase (HSV-tk) by transduced neural stem cells, wherein the anti-tumor activity observed is effected by gap junction-mediated bystander cell killing.

Miletic *et al.* (2007) demonstrate the principle of glioma bystander killing by tumor infiltrating hematopoietic progenitor cells. A bone marrow-derived, highly proliferative subpopulation of mesenchymal stem cells showed glioma infiltrating properties and was used as a vehicle for gene therapy of experimental malignant glioma. A therapeutic effect was demonstrated *in vivo* by the bone marrow derived cells expressing thymidine kinase of herpes simplex virus (HSV-tk) through bystander-mediated glioma cell killing after gancylovir application.

Also Nakamizo *et al.* (2005) describe the migratory behaviour of human bone marrow-derived mesenchymal stem cells (hMSCs), which can integrate into human gliomas after intravascular or local delivery and, thus, can be employed as a vehicle for the delivery of therapeutic agents, such as interferon-β, to brain tumors. The authors also found that using non-secreting hMSCs, a shorter survival than in control animals was observed. It was concluded by the authors that for a therapeutic approach relatively pure populations of stem cells engineered to carry a therapeutic agent and free of populations of untransfected cells will be needed.

Thus, neural and mesenchymal stem cells have been discussed in the art as delivery vehicles for intra-tumoral delivery of therapeutic genes. However, these methods have the drawback that the cells need to be further modified by genetic engineering to obtain a population of cells expressing the desired therapeutically active agents. In addition, there are several important limitations in terms of large-scale production of these currently investigated cells for clinical application. Neuronal stem cells are isolated from fetal or adult brain tissue and form a mixed cell population with limited number of passaging (Ben-Hur, 2008). Mesenchymal stem cells. are much easier to access e.g. from the bone marrow, but also have a low passaging capacity, with a maximum of up to 5-30 population doublings and change their cell surface marker expression over a longer culture time period (Uccelli et al., 2007). Furthermore, both neuronal and mesenchymal stem cells have immunosuppressive effects, which has been employed therapeutically in preventing autoimmune diseases and transplantation rejection (Ben-Hur (2008); Uccelli *et al.* (2007)), but which suppresses the host immune response to the glioma.

The technical problem underlying the present invention is thus the provision of alternative and/or improved means and methods for treating malignant neoplasms of the central nervous system.

The solution to this technical problem is achieved by providing the embodiments characterised in the claims.

Accordingly, the present invention relates to microglial precursor cells for use in the treatment of a malignant neoplasm of the central nervous system.

In accordance with the present invention, the term "microglial precursor cells" relates to a population of cells comprising partially differentiated cells, derived from myeloid precursor cells and capable of further differentiating into microglial cells. Myeloid precursor cells are characterized by the expression of the transcription factor PU.1 as a marker (Iwasaki *et al.* (2005). Microglia are further characterized by a ramified morphology with processes interdigitating with other glial cells and neurons and surveying their local (Ransohoff and Perry (2009)). Furthermore, upon transplantation of microglial precursor cells into tissues comprising neurons and astrocytes, they integrate into these tissues as microglia (Tsuchiya *et al.,* (2005); Napoli, (2008)). In addition, upon contact of microglial precursors with neurons and/or astrocytes or addition of growth factors such as macrophage colony-stimulating factor, they can transform into microglia (Liu *et al.* (1994)). The population of "microglial precursor cells" may comprise cells at different stages of differentiation between myeloid precursor cells and microglia. Thus, also fully differentiated microglial cells may be comprised in the population of microglial precursor cells. Non-differentiated stem cells as well as neural or mesenchymal stem ceiis are not comprised in the term "microglial precursor cells". Preferably, the microglial precursor cells are a population comprising at least 70% of cells expressing the markers CD45, CD11b, CD11c, CD14, CD16, CD36, CD68, integrin-α4, integrin-β1, CX3CR1, TREM2 and DAP12 and being inducible to express tumor necrosis factor-α, interleukin-1β, nitric oxide synthase-2, CX3CL1, CCL2, CXCL9 and/or CXCL10 (Napoli, (2008)). More preferably, at least 80%, such as at least 90%, at least 95% and most preferably 100% of the cells express the markers CD45, CD11b, CD11c, CD14, CD16, CD36, CD68, integrin-α4, integrin-β1, CX3CR1, TREM2 and DAP12 and are inducible to express tumor necrosis factor-α, interleukin-1β, nitric oxide synthase-2, CX3CL1, CCL2, CXCL9 and/or CXCL10.

Microglia are the resident immune cell types of the central nervous system (CNS). Microglia populate and invade the CNS during neuroectodermal development. Under pathological conditions such as infectious diseases, stroke, or neurodegenerative processes, microglia become activated, migrate to and within the lesion site, release a wide range of soluble factors that include cytotoxins, neurotrophins and immunomodulatory factors and clear cellular debris by phagocytosis.

The term "malignant neoplasm of the central nervous system" as used in accordance with the present invention relates to a tumor mass resulting from the abnormal proliferation of cells within the central nervous system. Malignant neoplasm of the central nervous system include malignant neoplasms of the brain, the spinal cord as well as other parts of the central nervous system. As classified by the World Health Organization, malignant neoplasms of the brain include for example malignant neoplasms of the cerebrum, malignant neoplasms of the frontal lobe, the temporal lobe, the parietal lobe, the occipital lobe, the cerebral ventricle excluding the fourth ventricle, malignant neoplasms of the cerebellum or the brain stem. Malignant neoplasms of the spinal cord and other parts of the central nervous system include, according to the classification of the World Health Organization, for example malignant neoplasms of the spinal cord, the cauda equina, the olfactory nerve, the optic nerve, the acoustic nerve as well as other cranial nerves (ICD 10 International Statistical Classification of Diseases And Related Health Problems: Tenth Revision: 2nd edition, vol. 1 to 3; World Health Organization 2004).

In accordance with the present invention it was surprisingly found that injection of microglial precursor cells into gliomas not only resulted in invasion and spreading of these cells within the tumor tissue without being rejected, but also had a clear effect on tumor size. Tumor size was reduced by 10-fold in mice injected with the microglial precursor cells. In contrast, when using bone marrow derived cultured mesenchymal cells, no reduction in tumor size was observed. Thus, in accordance with the present invention it was surprisingly shown that glioma size can be successfully reduced by using microglial precursor cells on their own. The effect on glioma size does not require the use of these cells as a delivery vehicle for therapeutic agents, thus obviating the need for extensive genetic manipulation of the cells. Furthermore, as the success of the treatment does not depend on the use of therapeutic agents, such as for example chemotherapeutic agents, potential side effects associated with the use of such agents can be prevented.

In addition, the microglial precursor cells used in accordance with the present invention also overcome the above discussed limitations encountered when using neural or mesenchymal stem cells, i.e. microglial precursor cells can be obtained from induced pluripotent stem cells, embryonic stem cells or other pluri- or multipotent stem or precursor cells, which have unlimited proliferative potential.

Preferably, the microglial precursor cells of the invention are comprised in a pharmaceutical composition, optionally further comprising a pharmaceutically acceptable carrier, excipient. and/or diluent.

The term "pharmaceutical composition", as used herein, relates to a composition for administration to a patient, preferably a human patient. The pharmaceutical composition of the invention comprises at least the microglial precursor cells of the invention. Preferably, the composition may be in liquid form and may be, inter alia, (a) solution(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, isotonic water, emulsions, such as oil/water emulsions, various types of wetting agents, sterile solutions etc. Compositions comprising such carriers can be formulated by well known conventional methods. The pharmaceutical composition may be administered locally or systemically, i.e. administration may be effected for example by intravenous, intratumoral or intraperitoneal administration. Preparations for parenteral administration include sterile aqueous or non-aqueous solutions and suspensions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

It is particularly preferred that said administration is carried out by injection and/or delivery, e.g., to a site in the bloodstream such as a brain artery, into the cerebro-spinal liquor or directly into the respective malignant neoplasm. The compositions of the invention may also be administered directly to the target site, e.g., by biolistic delivery to an external or internal target site, like the brain. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Administration may be carried out once in a single dose or as repeat treatments. Microglial precursor cells may be present in amounts between 1x10⁴ to 1x10⁷/kg body weight per dose, such as for example in amounts between 1x10⁵ and 1x10⁷/kg body weight per dose. Preferably, microglial precursor cells may be present in amounts between 1x10⁶ to 1x10⁷/kg body weight per dose; however, doses below or above this exemplary range are envisioned, especially considering the aforementioned factors. Progress can be monitored by periodic assessment.

It is particularly preferred that said pharmaceutical composition comprises further agents known in the art to antagonize malignant neoplasms of the central nervous system. Since the pharmaceutical preparation of the present invention relies on the above mentioned microglial precursor cells, it is preferred that the further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The present invention furthermore relates to a method of treating a malignant neoplasm of the central nervous system comprising administering a pharmaceutically effective amount of microglial precursor cells to a subject in need thereof.

In a preferred embodiment, the microglial precursor cells of the invention have admixed thereto or associated in a separate container chemo-attractants for cytotoxic immune cells and/or immunostimulatory substances for microglial precursors.

The term "chemo-attractants for cytotoxic immune cells" as used herein refers to substances capable of attracting cytotoxic immune cells, such as for example natural killer (NK) cells, cytotoxic T. cells or cytotoxic lymphocytes. Non-limiting examples of chemo-attractants for cytotoxic immune cells include the chemokine (C-X3-C motif) ligand 1 (CX3CL1; Xin *et al.* (2005)), the chemokine (C-X-C motif) ligand 9 (CXCL9) and the chemokine (C-X-C motif) ligand 10 (CXCL10). A variety of methods are known in the art for determining whether a substance acts as a chemo-attractant for cytotoxic immune cells, such as for example measuring chemotaxis using a migration assay or determining directed migration towards a chemokine gradient, using for example the Chemicon QCM™ Chemotaxis Migration Assay.

In accordance with the present invention, the term "immunostimulatory substances for microglial precursors" refers to substances that have the capacity to act immunostimulatory on microglial precursors, for example by inducing the gene transcription of pro-inflammatory cytokines and reactive oxygen species by said cells.

Non-limiting examples of immunostimulatory substances for microglial precursors include agonists of Toll-like receptors (TLR) or agonists of RIG-I-like receptors (RLR) as well as interferons, such as interferon type I (interferon-α and interferon-β) and interferon type II (interferon-γ). A variety of methods for determining whether a substance is an immunostimulatory substances for microglial precursors are known in the art, such as for example measuring the gene expression of tumor necrosis factor- α (TNF-α), interleukin-1β (IL1β) and nitric oxide synthase-2 (NOS2) by real-time PCR before and after stimulation with the potential immunostimulatory substance, wherein an up-regulation of these genes is an indication that the tested substance acts immunostimulatory on microglial precursors. As another exemplary method, the amount of these cytokines may be determined using ELISA assays in the cell culture supernatant.

Agonists of Toll-like receptors include Pam3Cys-SK4 (ligand for TLR1/2), poly(I:C) (TLR3 ligand), purified LPS (TLR4 ligand), R848 (TLR7 ligand) and CpG-oligonucleotides (TLR9 ligands) (Grauer *et al.* (2008)).

The use of such chemo-attractants for cytotoxic immune cells as well as of immunostimulatory substances for microglial precursors may aid the antitumoral effect observed by providing the microglial precursor cells of the present invention by further stimulating the natural immune responses of the host.

In a preferred embodiment, the microglial precursor cells are characterised by the expression of CD45, CD11b, CD11c, CD14, CD16, CD36, CD68, integrin-α4, integrin-β1, CX3CR1, TREM2 and DAP12.

All of these molecules characterising microglial precursor cells are defined in accordance with the present invention in the same manner as known in the prior art and the common general knowledge of the skilled person.

"CD45", as used herein, refers to cluster of differentiation 45 and is a membrane tyrosine phosphatase that is used as a marker to distinguish cells of the hematopoietic lineage from the endothelial lineage. CD45 is uniformly distributed in plasma membrane and constitutes up to 10% of the molecules on the surface of expressing cells (Ford *et al.* (1995)).

in accordance with the present invention, "CD11b" relates to cluster of differentiation 11b, a subunit of Mac-1, which is a complement receptor ("CR3") consisting of CD11b and CD18.

"CD11c", in accordance with the present invention, refers to cluster of differentiation 11c, a type I transmembrane protein found at high levels on most human dendritic cells, but also on monocytes, macrophages, neutrophils and some B cells (Ford *et al.* (1995)).

In accordance with the present invention, "CD14" refers to cluster of differentiation 14, which is a membrane protein found mainly on macrophages which binds to bacterial lipopolysaccharide.

"CD16", as used herein, refers to cluster of differentiation 16. CD16 is also termed FcγRIII and is a low-affinity Fc receptor for ImmunoglobulinG. It is mainly found on NK cells, macrophages, and neutrophils (Ulvestad *et al.* (1994).

"CD36", or cluster of differentiation 36, in accordance with the present invention, refers to the platelet glycoprotein IV or IIIb (GP IV / GP IIIb).

In accordance with the present invention, "CD68", refers to cluster of differentiation 68, a 110 kDa highly glycosylated transmembrane protein which is mainly located in lysosomes and is a marker for monocytes/macrophages.

The term "integrin-α4", as used herein, refers to the α4 integrin subunit expressed on the cell membrane as a heterodimer non-covalently associated with the β1 or the β7 integrin chains. α4 integrins have a key function in the adhesion interaction between stem/progenitor cells and the stromal microenvironmental cells and their matrix within the bone marrow.

In accordance with the present invention, "integrin-β1", also known as ITGB1 or CD29, is an integrin unit associated with very late antigen receptors.

The term "CX3CR1" as used herein, refers to the only member of the CX3C sub-family of chemokine receptors. This receptor binds the chemokine CX3CL1 (described above), which is also known as neurotactin or fractalkine. Expression of this receptor is mainly associated with macrophages and monocytes (Jung *et al.* (2000)).

"TREM2", in accordance with the present invention, refers to the triggering receptor expressed on myeloid cells 2, and is believed to have a role in chronic inflammation and the stimulation of production of constitutive rather than inflammatory chemokines and cytokines (Schmid *et al.* (2002); Takahashi *et al.* (2005)). TREM2 forms a receptor signaling complex with TYROBP and triggers activation of the immune responses in macrophages and dendritic cells.

In accordance with the present invention, "DAP12", also termed TYROBP, encodes a transmembrane signaling polypeptide which contains an immunoreceptor tyrosine-based activation motif (ITAM) in its cytoplasmic domain (Roumier *et al.* (2004). The encoded protein may associate with the killer-cell inhibitory receptor (KIR) family of membrane glycoproteins and may act as an activating signal transduction element. This protein may bind zeta-chain (TCR) associated protein kinase 70kDa (ZAP-70) and spleen tyrosine kinase (SYK) and play a role in signal transduction, bone modeling, brain myelination, and inflammation. Its putative receptor is TREM2.

As microglial precursor cells have been shown to express the above mentioned markers (Napoli (2008); Schmid *et al*. (2002); Takahashi *et al.* (2005); Roumier *et al.* (2004); Jung *et al.* (2000); Ford *et al.* (1995); Ulvestad *et al.* (1994)), said marker molecules may be used to confirm the identity of cells as microglial precursor cells.

In another preferred embodiment, the microglial precursor cells are characterised by being inducible to express tumor necrosis factor-α, interleukin-1β, nitric oxide synthase-2, CX3CL1, CCL2, CXCL9 and/or CXCL10.

Also these inducible molecules characterising microglial precursor cells are defined in accordance with the present invention in the same manner as known in the prior art and the common general knowledge of the skilled person.

"Tumor necrosis factor-α (TNF-α)", in accordance with the present invention, is a cytokine involved in systemic inflammation and is a member of a group of cytokines that stimulate the acute phase reaction. TNF-α primarily regulates immune cells, but is also able to induce apoptotic cell death, to induce inflammation, and to inhibit tumorigenesis and viral replication. Dysregulation and, in particular, overproduction of TNF-α have been implicated in a variety of human diseases, as well as cancer. TNF-α is produced mainly by macrophages, but also by a broad variety of other cell types including lymphoid cells, mast cells, endothelial cells, cardiac myocytes, adipose tissue, fibroblasts, and neuronal tissue. Large amounts of TNF-α are released in response to lipopolysaccharide, other bacterial products, and interleukin-1 (IL-1).

In accordance with the present invention, "interleukin-1β (IL-1β)" refers to a cytokine and is a member of the interleukin-1 cytokine family. Interleukin-1β is produced by activated macrophages as a proprotein, which is proteolytically processed to its active form by caspase-1 (CASP1/ICE). Interleukin-1β is an important mediator of the inflammatory response, and is involved in a variety of cellular activities, including cell proliferation, differentiation, and apoptosis.

As used herein, "nitric oxide synthase-2 (NOS2)" belongs to a family of enzymes that carry out a 5'-electron oxidation of L-arginine with the aid of tetrahydro-biopterin. NOS enzymes contribute to the transmission between neurons, to the immune system and to dilating blood vessels, They do so by synthesis of nitric oxide (NO) from the terminal nitrogen atom of L-arginine in the presence of NADPH and dioxygen (O₂). Induction of the NOS2 usually occurs in an oxidative environment, and thus high levels of nitric oxide have the opportunity to react with superoxide leading to peroxynitrite formation and cell toxicity. These properties are believed to define the roles of NOS2 in host immunity, enabling its participation in antimicrobial and anti-tumor activities as part of the oxidative burst of macrophages.

"CX3CL1", as used herein, refers to the chemokine (C-X3-C motif) ligand 1 (CX3CL1). CX3CL1 is a large cytokine of 373 amino acids, which contains multiple domains. It is the only known member of the CX3C chemokine family. CX3CL1 is also commonly known under the names fractalkine (in humans) and neurotactin (in mice). Soluble CX3CL1 potently chemoattracts T cells and monocytes, while the cell-bound chemokine promotes strong adhesion of leukocytes to activated endothelial cells, where it is primarily expressed. CX3CL1 elicits its adhesive and migratory functions by interacting with the chemokine receptor CX3CR1.

In accordance with the present invention, "CCL2" refers to the chemokine (C-C motif) ligand 2. CCL2 is a small cytokine belonging to the CC chemokine family that is also known as monocyte chemotactic protein-1 (MCP-1). CCL2 recruits monocytes, memory T cells, and dendritic cells to sites of tissue injury and infection. Cell surface receptors that bind CCL2 are CCR2 and CCR4.

"CXCL9", as used throughout the present invention, refers to chemokine (C-X-C motif) ligand 9. CXCL9 is a small cytokine belonging to the CXC chemokine family that is also known as monokine induced by gamma interferon (MIG). CXCL9 is a T-cell chemoattractant, which is induced by IFN-γ. CXCL9 elicits its chemotactic functions by interacting with the chemokine receptor CXCR3.

"In accordance with the present invention, "CXCL10" refers to chemokine (C-X-C motif) ligand 10. CXCL10 is a small cytokine belonging to the CXC chemokine family that is also known as 10 kDa interferon-gamma-induced protein (γ-IP10 or IP-10). CXCL10 is secreted by several cell types in response to IFN-γ. These cell types include monocytes, endothelial cells and fibroblasts. CXCL10 has been attributed several roles, such as chemoattraction for monocytes/macrophages, T cells, NK cells, and dendritic cells, promotion of T cell adhesion to endothelial cells, antitumor activity, and inhibition of bone marrow colony formation and angiogenesis. This chemokine elicits its effects by binding to the cell surface chemokine receptor CXCR3.

It has been shown that microglial precursor cells can be induced to express the above mentioned factors (Napoli. (2008); Carter *et al.* (2007); Hughes *et al.* (2002); Gourmala *et al.* (1997)). Thus, in accordance with this embodiment, the microglial precursor cells are characterized by being inducible to express tumor necrosis factor-α (TNF-α), interleukin-1β (IL-1β), nitric oxide synthase-2 (NOS2), CX3CL1, CCL2, CXCL9 and/or CXCL10, Induction is preferably achieved by an immunostimulatory substance for microglial precursors, as described above.

In another preferred embodiment, the microglial precursor cells are derived from induced pluripotent stem (iPS) cells, embryonic stem cells or other pluri- or multipotent stem or precursor cells.

"Induced pluripotent stem (iPS) cells", in accordance with the present invention, are pluripotent stem cell derived from a non-pluripotent cell, typically an adult somatic cell, by inducing a "forced" expression of certain genes. Induced pluripotent stem cells are identical to natural pluripotent stem cells, such as embryonic stem cells in many respects, such as the expression of certain stem cell genes and proteins, chromatin methylation patterns, doubling time, embryoid body formation, teratoma formation, viable chimera formation, and potency and differentiability. Induced pluripotent stem cells are an important advancement in stem cell research, as they allow researchers to obtain pluripotent stem cells without the use of embryos (Nishikawa *et al.* (2008)). The induced pluripotent stem cells may be obtained from any adult somatic cells, preferably from fibroblasts, e.g. from skin tissue biopsies.

The term "embryonic stem cells", as used throughout the present invention, refers to stem cells derived from the inner cell mass of an early stage embryo known as a blastocyst. Embryonic stem (ES) cells are pluripotent, i.e. they are able to differentiate into all derivatives of the three primary germ layers: ectoderm, endoderm, and mesoderm. Recent advances in embryonic stem cell research have led to the possibility of creating new embryonic stem cell lines without destroying embryos, for example by using a single-cell biopsy similar to that used in preimplantation genetic diagnosis (PGD), which does not interfere with the embryo's developmental potential (Klimanskaya *et al.* (2006)).

The term "other pluri- or multipotent stem or precursor cells", in accordance with the present invention, relates to a cell type having the capacity for self-renewal, an ability to go through numerous cycles of cell division while maintaining the undifferentiated state, and the potential of differentiation, i.e. the capacity to differentiate into specialized cell types. In the strictest sense, this requires stem cells to be either totipotent or pluripotent - to be able to give rise to any mature cell type, although multipotent, oligopotent or unipotent progenitor cells are sometimes referred to as stem cells. Pluripotent stem cells are the descendants of totipotent cells and can differentiate into nearly all cells, i.e. cells derived from any of the three germ layers. Totipotent stem cells can differentiate into embryonic and extraembryonic cell types. Multipotent stem cells can differentiate into a number of cells, but only those of a closely related family of cells. Oligopotent stem cells can differentiate into only a few cells, such as lymphoid or myeloid stem cells (Wagers and Weissman (2004). Examples of "other pluri- or multipotent stem or precursor cells" include pluripotent stem cells, multipotent stem or precursor cells and without being limiting, also PU.1 expressing myeloid precursor cells derived from hematopoietic stem cells. The PU.1 expressing myeloid precursors or hematopoietic stem cells may be obtained from bone marrow, yolk sac, fetal liver or the developing central nervous system. In this respect it is important to note that neural or mesenchymal stem cells, as used in the prior art, are not encompassed by this term, as these cells are derived from precursor cells not expressing PU.1 and are not capable of differentiating into microglial cells.

In another preferred embodiment, the malignant neoplasm of the central nervous system is a malignant neoplasm of the brain.

Thus, in this embodiment the malignant neoplasm of the brain may be, for example, a malignant neoplasm of the cerebrum with the exception of lobes and ventricles, a malignant neoplasm of the frontal lobe, the temporal lobe, the parietal lobe, the occipital lobe, the cerebral ventricle excluding the fourth ventricle or a malignant neoplasm of the cerebellum or the brain stem.

In a more preferred embodiment, the malignant neoplasm of the brain is a glioma.

In accordance with the present invention, the term "glioma" refers to a tumor that arises from glial cells. Gliomas are classified by cell type, by grade, and by location. Classification by cell type is generally according to the specific type of cell the glioma most closely resembles. The main types of gliomas are ependymomas, that resemble ependymal cells; astrocytomas (with glioblastoma multiforme being the most common astrocytoma), that resemble astrocytes; oligodendrogliomas, that resemble oligodendrocytes and mixed gliomas, such as oligoastrocytomas, that contain cells from different types of glia. As the skilled person knows, oligoastrocytomas may also contain glial precursor or stem cells. Gliomas are further categorised according to their grade, which is determined by pathologic evaluation of the tumor. The most common grading system is the World Health Organization (WHO) grading system for astrocytoma. The WHO system assigns a grade from 1 to 4, with 1 being the least aggressive and 4 being the most aggressive. Gliomas may also be classified according to whether they are above or below a membrane in the brain called the tentorium. The tentorium separates the cerebrum, above, from the cerebellum, below. Thus, the classification is supratentorial when the glioma is above the tentorium, in the cerebrum, which is the case mostly in adults and it is infratentorial when the glioma is below the tentorium, in the cerebellum, which occurs mostly in children.

In a further more preferred embodiment, the glioma is selected from astrocytoma and oligodendroglioma.

Preferably, the astrocytome or oligodendroglioma is selected from anaplastic astrocytoma (WHO grade 3 astrocytoma), glioblastoma multiforme (WHO grade 4 astrocytoma), anaplastic oligodendroglioma (WHO grade 3 oligodendroglioma) and anaplastic oligoastrocytoma (WHO grade 3 oligoastrocytoma/ anaplastic mixed glioma).

All of the diseases described herein are well known to the skilled person and are defined in accordance with the prior art and the common general knowledge of the skilled person.

The figures show:

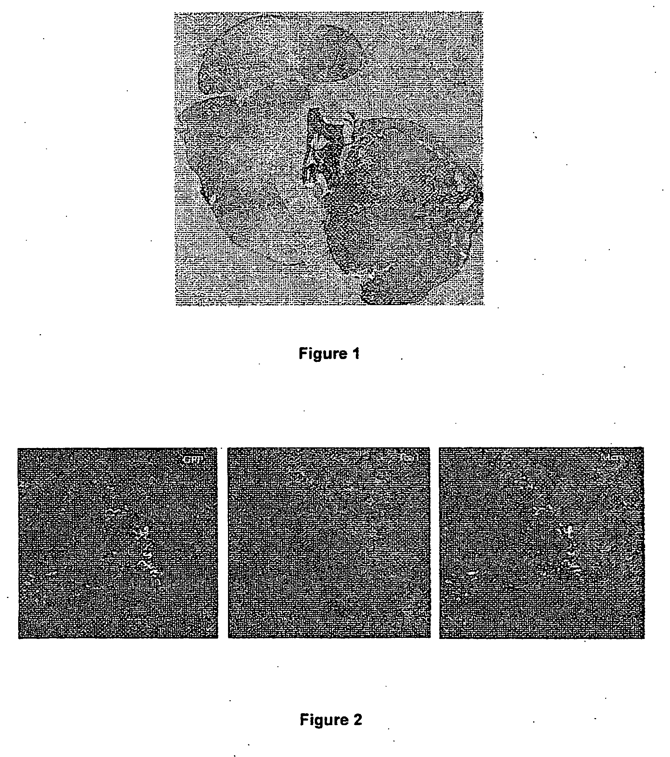

**Figure 1****.** Composed histological section of a solid tumor (glioma) at 3 weeks after intracerbral injection of 1000 SMA 560 cells in the brain of VM/DK mice.
**Figure 2****.** Glioma invasion by GFP-transduced ES cell-derived microglial precursor cells. ES cell-derived microglial precursor cells were injected 5 days after establishment of tumors. At 16 days after transplantation, the microglial precursor cells invaded the entire tumor tissue. They appeared to be enriched at the border between the tumor and the normal brain tissue. Microglial cells were double-labelled with antibodies directed against Iba1.

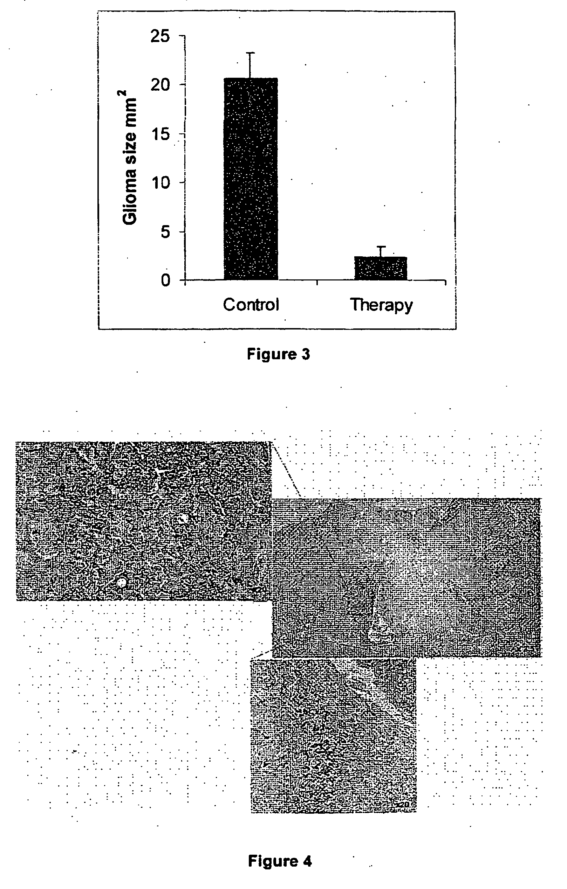

**Figure 3****.** Reduced glioma diameter after intratumoral injection of ES cell-derived microglial precursor cells. Injection of 5 x 10⁵ microglial precursor cells or PBS was performed at 5 days after tumor induction. Tumor size was analysed at 16 days after therapy with ES cell-derived microglial precursor cells. n=6 control and n=9 therapy group.
**Figure 4****.** Detection of NK1.1 positive cells in the microglial precursor cell-treated tumor residuum.

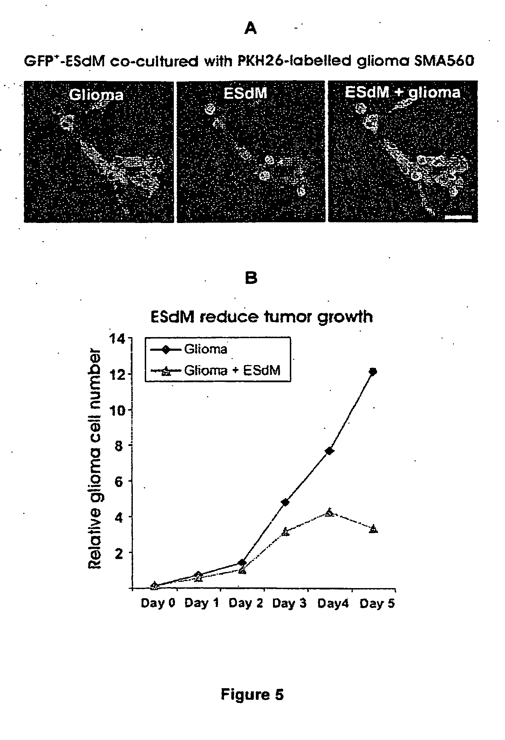

**Figure 5****.** A. Coculture of GFP-transduced ESdM and the glioma cell line SMA 560 labeled with the red membrane fluorescent dye PKH26. Cellular interactions between ESdM and glioma cells were detected by confocal microscope in a coculture system. B. ESdM were cocultured with glioma cells at a ratio of 1:1. Coculture with ESdM resulted in a reduced glioma cell number.

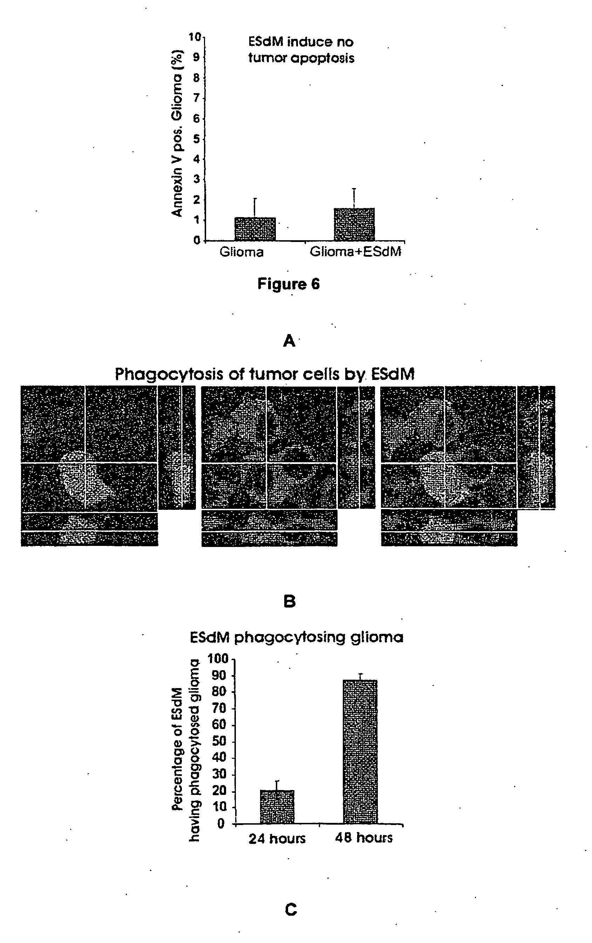

**Figure 6****.** Glioma cells were cocultured with GFP-transduced ESdM (Glioma+ESdM) or cultured alone (Glioma). The percentage of apoptotic cells was determined after 48 hours by labeling the cells with an AnnexinV linked fluorescent dye. The percentage of apoptotic glioma cells was independent on the presence or absence of ESdM.

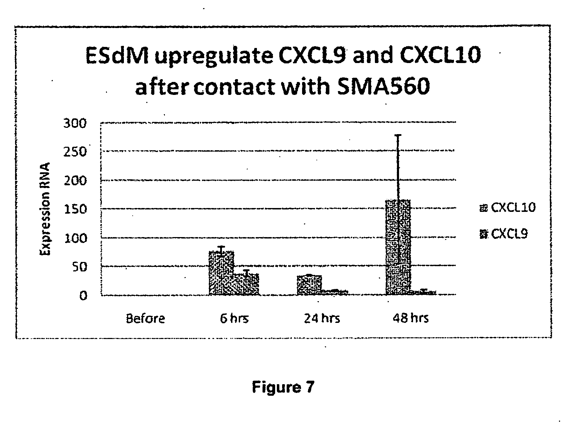

**Figure 7****.** Glioma cells labeled with a red fluorescent membrane dye were cocultured with GFP-transduced ESdM. A. Confocal images were obtained demonstrating the uptake of red labeled glioma membranes into lysosomal-like structures of the ESdM. Left image: GFP+ ESdM. Middle image: red labelled glioma membranes. Right image: merger. B. The percentage of ESdM showing phagocytosis of glioma membranes was quantified after 24 and 48 hours of co-culture. The majority of ESdM phagocytosed glioma cells at 48 hours after co-culture. C. Analyzation for gene transcription of chemokines by real-time RT-PCR ESdM cocultured with glioma showed increased gene transcription of the NK-cell attracting chemokines CXCL9 and CXCL10.

The examples illustrate the invention.

### Example 1: Microglial precursors obtained from mouse embryonic stem cells (ESdM).

Murine ES cell lines of C57BL/6 or 129 mouse have been differentiated *in vitro* into microglial precursors using a modified five-step method originally designed to obtain neurons (Lee et al., 2000). Undifferentiated ES cell clusters (Stage 1) were trypsinized and cultured for 2 days on gelantine-coated tissue culture plates in the presence of LIF (Chemicon) containing ES cell medium consisting of Dulbecco's modified Eagle's medium high glucose (DMEM, Invitrogen) supplemented with 15% fetal bovine serum (FBS, Invitrogen), 2 mM L-alanin-L-glutamine, 4 mM glutamine, 0.1 mM nonessential amino acids, 1 mM sodium pyruvate (all from Invitrogen), and 0.1 mM 2-mercaptoethanol (Chemicon). To induce embryoid body formation (Stage 2), cells were dissociated into a single cell suspension and plated on non-adherent bacterial culture dishes (BD Falcon) at a density of 2-2.5 x 10⁴ cells/cm² in the medium described above, but without LIF. After 3-4 days, the resulting EBs were plated on gelatine-coated dishes. After 24-48 hours of culture, selection and expansion of nestin positive cells was initiated with DMEM/F12 medium (Invitrogen) supplemented with 5 µg/mL insulin (Sigma), 30 nM sodium selenite (Sigma), 50 µg/mL transferrin (Millipore), and 5 µg/mL fibronectin (Sigma) (Stage 3). After 6 days of selection, expansion of cells was initiated in N2 medium consisting of DMEM/F12 medium supplemented with 1% N2 supplement (Invitrogen), 0.048 mM L-glutamine (Invitrogen). Proliferation of cells was observed 21 days after removal of growth factors. The cells were mechanically isolated and seeded in N2 medium containing 2% fetal bovine serum or 2% serum replacement factors. For further passaging, cells were detached with a cell scraper or trypsinized with 0.05% Trypsin/EDTA (Invitrogen). Cells were splitted twice a week at confluency and kept in 5% CO2 in N2 medium containing 5% FBS at 37°C. The phenotype of the cells was confirmed by flow cytometry analysis, immunohistochemistry and functional assays.

### Example 2: Intratumoral injection of microglial precursors into experimental gliomas

Experimental gliomas were induced in a syngenic animal model to avoid immune reaction against allo-antigens expressed by the tumor. The most widely syngenic tumor model is the astrocytome line SMA 560. The murine astrocytoma line SMA 560 was established from a mouse strain (VM/Dk), which spontaneously develops brain tumors in 1-3%. The SMA 560 cells have a high proliferation rate and develop solid gliomas after intracerebral injection. Injection of 1000 SMA 560 cells was performed unilaterally in adult VM/Dk mice. After 3 weeks, mice showed regularly gliomas with a diameter of 4-5 mm (Fig. 1).

ES cell-derived microglial precursor cells as described in Example 1 were lentivirally transduced with GFP to allow visualization of cells after intragliomal injection. In total, 5x10⁵ microglial precursor cells were injected at 5 days after tumor transplantation into the already established glioma. The transplanted microglial precursor cells invaded and spread within the tumor tissue. At 16 days after injection, the GFP-transduced microglial precursor cells were distributed all over the tumor (Fig. 2). At this time point, the microglial precursor cells were not rejected although they had a different haplotype (C57BL6) and expressed the foreign antigen GFP.

The microglial precursor cells had a clear effect on the tumor size. 3 weeks after glioma induction, mice were killed and analyzed by immunohistochemistry. The tumor size was determined after serial sectioning of the brain tissue. The maximum glioma size detected in serial sections of the total brain was reduced from 20.6 +/-2.7 mm² to 2.3 +/- 1.1. mm² by the injection of ESdM (Fig. 3). On the other hand, transplantation of bone marrow derived cultured mesenchymal cells did not result in an observable reduction in tumor size, indicating that ESdM are specifically reducing glioma size.

### Example 3: Detection of NK1.1 positive cells in the ESdM-treated tumor residuum.

Immunohistochemical staining for CD4 and CD8 did not show any evidence for increased infiltration of CD4+ or CD8+ lymphocytes. However, staining with an NK1.1 specific antibody resulted in labelling of many positive cells localised in the residuum of the tumor (Fig. 4). The NK1.1-positive cells were not detected in the control glioma after injection of PBS and did not overlap with the transplanted GFP-positive cells, indicating that the NK1.1-positive cell were attracted by the ESdM.

### Example 4: In vitro experiments with murine ESdM and glioma

In vitro experiments were performed to study the interaction between ESdM and the glioma cell line SMA560. Cultured ESdM were visualized by lentiviral transduction with GFP and glioma cells were labeled with the red membrane fluorescent dye PKH26 (Fig. 5A). Direct cell-cell contact between ESdM and glioma cells was observed under the fluorescence microscope (Fig. 5A).

Next, the effect of ESdM on tumor growth and glioma cell number was analyzed by counting the fluorescently labeled glioma cells under the microscope. ESdM caused a reduction of the glioma cell number starting from day 3 after co-culture (Fig. 5B). At day 4 and 5 the glioma cell number was clearly reduced by the ESdM (Fig. 5B). The incubation of the supernatant of cultured ESdM with glioma cells did not reduce tumor growth and glioma cell number, indicating that direct contact between ESdM and glioma was required.

In principal, microglia can reduce the cell number of target cells by production of cytotoxic factors (induction of apoptosis) or by phagocytic removal of the target cells. However, no induction of glioma apoptosis by ESdM was observed (Fig. 6). The number of AnnexinV positive glioma cells as an early sign of apoptosis was not changed after coculture with ESdM.

Then, the clearance of glioma through phagocytosis by ESdM was analysed. Glioma cells were labeled with a red fluorescent membrane dye and cocultured with GFP-transduced ESdM. Confocal images were obtained 48 hours after coculture (Fig. 7). Three-dimensional reconstruction of confocal images demonstrated the uptake of red labeled glioma cells into lysosomal-like structures of the ESdM (Fig. 7A) The percentage of ESdM showing phagocytosed red fluorescent labeled glioma membranes was quantified after 24 and 48 hours of coculture (Fig. 7B). The majority of ESdM showed phagocytosis of glioma cells, indicating that ESdM reduced the glioma cell number by phagocytic clearance.

Clearance of target structures can be executed by phagocytes silently (e.g. clearance of apoptotic cells) or can be associated with recruitment of additional immune cells by release of chemokines (e.g. clearance of microbes). Since the invasion of NK1.1+ cells into glioma after intra-tumoral transplantation of ESdM was observed, gene transcription of the immune cell attracting chemokines was analyzed (Fig. 7C). Therefore, GFP-transduced ESdM were cocultured with glioma for distinct time periods, then sorted by flow cytometry and analyzed for gene transcription of chemokines by real-time PCR. Data demonstrated that ESdM cocultured with glioma showed increased gene transcription of the NK cell attracting chemokines CXCL9 and CXCL10 (Fig. 7C).

Therefore, ESdM phagocytosed glioma cells and thus initiated a reduction in tumor mass and the clearance of the tumor.

### References

Aboody, K. S., Brown, A., Rainov, N. G., Bower, K. A., Liu, S., Yang, W., Small, J. E., Herrlinger, U., Ourednik, V., Black, P. M., et al. (2000). Neural stem cells display extensive tropism for pathology in adult brain: evidence from intracranial gliomas. Proc Natl Acad Sci USA 97, 12846-12851.
Ajami, B., Bennett, J. L., Krieger, C., Tetzlaff, W., and Rossi, F. M. (2007). Local selfrenewal can sustain eNS microglia maintenance and function throughout adult life. Nat Neurosci 10,1538-1543.
Ben-Hur, T. (2008). Immunomodulation by neural stem cells. J Neural Sci 265,102-104.
Carpentier, A. F., and Meng, Y. (2006). Recent advances in immunotherapy for human glioma. Curr Opin Oncol 18,631-636.
Carter SL, Müller M, Manders PM, Campbell IL. (2007). Induction of the genes for Cxcl9 and Cxcl10 is dependent on IFN-gamma but shows differential cellular expression in experimental autoimmune encephalomyelitis and by astrocytes and microglia in vitro. Glia. 55(16):1728-39
Ford AL, Goodsall AL, Hickey WF, Sedgwick JD. (1995). Normal adult ramified microglia separated from other central nervous system macrophages by flow cytometric sorting. Phenotypic differences defined and direct ex vivo antigen presentation to myelin basic protein-reactive CD4+ T cells compared. J Immunol. 154(9):4309-21.
Gourmala NG, Buttini M, Limonta S, Sauter A, Boddeke HW. (1997). Differential and time-dependent expression of monocyte chemoattractant protein-1 mRNA by astrocytes and macrophages in rat brain: effects of ischemia and peripheral lipopolysaccharide administration. J Neuroimmunol. 74(1-2):35-44.)
Grauer, O.M., Molling, J.W., Bennink, E., Toonen, L.W.J., Sutmuller, R.P.M., Nierkens, S. and Adema, G.J (2008). TLR Ligands in the Local Tratment of Established Intracerebral Murine Gliomas. J. Immunol 181, 6720-6729.
Herrlinger, U., Wojciechowski, C., Sena-Esteves, M., Aboody, K. S., Jacobs, A. H., Rainov, N. G., Snyder, E. Y., and Breakefield, X. O. (2000). Neural precursor cells for delivery of replication-conditional HSV-1 vectors to intracerebral gliomas. Mol Ther 1, 347-357.
Huang, D., Shi, F. D., Jung, S., Pien, G. C., Wang, J., Salazar-Mather, T. P., He, T. T., Weaver, J. T., Ijunggren, H. G., Biron, C. A., at al. (2006). The neuronal chemokine CX3CL1/fractalkine selectively recruits NK cells that modify experimental autoimmune encephalomyelitis within the central nervous system. Faseb J 20, 896-905*.*
Hughes PM, Botham MS, Frentzel S, Mir A, Perry VH. (2002). Expression of fractalkine (CX3CL1) and its receptor, CX3CR1, during acute and chronic inflammation in the rodent CNS. Glia. 37(4):314-27.
Iwasaki, H., Somoza, C., Shigematsu, H., Duprez, E.A., iwasaki-Aral, J., Mizuno, S., Arinobu, Y., Geary, K., Zhang, P., Dayaram, T., Fenyus, M.L., Elf, S., Chan, S., Kastner, P., Huettner, C.S., Murray, R., Tenen, D.G. and Akashi, K. (2005). Distinctive and indispensable roles of PU.1 in maintenance of hematopoietic stem cells and their differentiation. Blood 106(5): 1590-1600.
Jung S, Aliberti J, Graemmel P, Sunshine MJ, Kreutzberg GW, Sher A, Littman DR. (2000). Analysis of fractalkine receptor CX(3)CR1 function by targeted deletion and green fluorescent protein reporter gene insertion. Mol Cell Biol. 20(11):4106-14.
Klimanskaya, I., Chung, Y., Becker, S., Lu, S.J., Lanza, R. (2006). Human embryonic stem cell lines derived from single blastomeres. Nature. 444(7118):481-5.
Liu W, Brosnan CF, Dickson DW, Lee SC. (1994). Macrophage colony-stimulating factor mediates astrocyte-induced microglial ramification in human fetal central nervous system culture, Am J Pathol. 145(1):48-53.
Mildner, A., Schmidt, H., Nitsche, M., Merkler, D., Hanisch, U. K., Mack, M., Heikenwalder, M., Bruck, W., Priller, J., and Prinz, M. (2007). Microglia in the adult brain arise from Ly-6ChiCCR2+ monocytes only under defined host conditions. Nat Neurosci 10, 1544-1553*.*
Miletic, H., Fischer, Y., Litwak, S., Giroglou, T., Waerzeggers, Y., Winkeler, A., Li, H., Himmelreich, U., Lange, C., Stenzel, W., et al. (2007). Bystander killing of malignant glioma by bone marrow-derived tumor-infiltrating progenitor cells expressing a suicide gene. Mol Ther 15,1373-1381.
Napoli, I. (2008). Establishment of Embryonic Stem Cell Derived Microglial Precursors and Application in an Animal Model of Alzheimer's Disease; PhD. thesis at the Faculty of Mathematics and Natural Sciences of the Rheinische Friedrich-Whilhelms University of Bonn.
Nakamizo, A., Marini, F., Amano, T., Khan, A., Studeny, M., Gumin, J., Chen, J., Hentschel, S., Vecil, G., Dembinski, J., et aL (2005). Human bone marrow-derived mesenchymal stem cells in the treatment of gliomas. Cancer Res 65, 3307 - 3318.
Nitta, T. (1998). Cytokine gene expression within the central nervous system. Cell Mol Neurobiol 18,703-708.
Ransohoff, R. M. (2007). Microgliosis: the questions shape the answers. Nat Neurosci 10,1507-1509.
Ransohoff, R.M. and Perry, V.H. (2009). Microglial physiology: unique stimuli, specialized responses. Annu Rev Immunol. 27:119-45.
Roumier A, Béchade C, Poncer JC, Smalla KH, Tomasello E, Vivier E, Gundelfinger ED, Triller A, Bessis A. (2004). Impaired synaptic function in the microglial KARAP/DAP12-deficient mouse. J Neurosci. 24(50):11421-8.
Schmid CD, Sautkulis LN, Danielson PE, Cooper J, Hasel KW, Hilbush BS, Sutcliffe JG, Carson MJ. (2002). Heterogeneous expression of the triggering receptor expressed on myeloid cells-2 on adult murine microglia. J Neurochem. 83(6):1309-20.
Takahashi K, Rochford CD, Neumann H. (2005). Clearance of apoptotic neurons without inflammation by microglial triggering receptor expressed on myeloid cells-2. J Exp Med. 201(4):647-57.
Tsuchiya, T., Park, K. C., Toyonaga, S., Yamada, S. M., Nakabayashi, H., Nakai, E., Ikawa, N., Furuya, M., Tominaga, A., and Shimizu, K. (2005). Characterization of microglia induced from mouse embryonic stem cells and their migration into the brain parenchyma. J Neuroimmunol 160, 210-218.
Uccelli, A., Pistoia, V., and Maretta, L. (2007). Mesenchymal stem cells: a new strategy for immunosuppression? Trends Immunol28, 219-226.
Ulvestad E, Williams K, Vedeler C, Antel J, Nyland H, Mørk S, Matre R. (1994). Reactive microglia in multiple sclerosis lesions have an increased expression of receptors for the Fc part of IgG. J Neurol Sci. 121(2):125-31.
Umemura, N., Saio, M., Suwa, T., Kitoh, V., Bai, J., Nonaka, K., Ouyang, G. F., Okada, M., Balazs, M., Adany, R., et al. (2008). Tumor-infiltrating myeloid-derived suppressor cells are pleiotropic-inflamed monocytes/macrophages that bear M1- and M2-type characteristics. J Leukoc Bioi 83, 1136-1144.
Wagers AJ, Weissman IL. (2004). Plasticity of adult stem cells. Cell. 116(5):639-48.
Weller, M., and Fontana, A. (1995). The failure of current immunotherapy for malignant glioma. Tumor-derived TGF-beta, T-cell apoptosis, and the immune privilege of the brain. Brain Res Brain Res Rev 21, 128-151.
Xin, H., Kikuchi, T., Andarini, S., Ohkouchi, S., Suzuki, T., Nukiwa, T., Huqun, Hagiwara, K., Honjo, T., and Saijo, V. (2005). Antitumor immune response by CX3CL1 fractalkine gene transfer depends on both NK and T cells. Eur J Immunol 35, 1371-1380.

## Claims

1. Microglial precursor cells for use in the treatment of a malignant neoplasm of the central nervous system.

2. The microglial precursor cells for use according to claim 1 , wherein the microglial precursor cells have admixed thereto or associated in a separate container chemoattractants for cytotoxic immune cells and/or immunostimulatory substances for microglial precursors.

3. The microglial precursor cells for use according to claim 1 or 2, wherein the microglial precursor cells are **characterised by** the expression of CD45, CD11b, CD11c, CD14, CD16, CD36, CD68, integrin-alpha4. integrin-betal, CX3CR1, TREM2 and DAP12.

4. The microglial precursor cells for use according to claim 1 or 3 , wherein the microglial precursor cells are **characterised by** being inducible to express tumor necrosis factor-α, interleuldn-1β, nitric oxide synthase-2, CX3CL1, CCL2, CXCL9 and/or CXCL10.

5. The microglial precursor cells for use according to any one of the preceding claims, wherein the microglial precursor cells are derived from induced pluripotent stem (iPS) cells, embryonic stem cells or other pluri- or multipotent stem or precursor cells.

6. The microglial precursor cells any one of claims 1 and 3 to 6 , wherein the malignant neoplasm of the central nervous system is a malignant neoplasm of the brain.

7. The microglial precursor cells claim 6, wherein the malignant neoplasm of the brain is a glioma.

8. The microglial precursor cells claim 7, wherein the glioma is selected from astrocytome and oligodendroglioma.

## Patentansprüche

1. Mikrogliale Vorläuferzellen für die Verwendung bei der Behandlung einer malignen Neoplasie des Zentralnervensystems.

2. Mikrogliale Vorläuferzellen für die Verwendung nach Anspruch 1, wobei den mikroglialen Vorläuferzellen chemische Anziehungsstoffe ("Chemoattractants") für zytotoxische Immunzellen und/oder immunstimulatorische Substanzen für mikrogliale Vorläuferzellen beigemischt oder in einem separaten Behälter assoziiert sind.

3. Mikrogliale Vorläuferzellen für die Verwendung nach Anspruch 1 oder 2, wobei die mikroglialen Vorläuferzellen von der Expression von CD45, CD11b, CD11c, CD14, CD16, CD36, CD68, Integrin-alpha4, Integrin-betal, CX3CR1, TREM2 und DAP12 gekennzeichnet sind.

4. Mikrogliale Vorläuferzellen für die Verwendung nach Anspruch 1 oder 3, wobei die mikroglialen Vorläuferzellen **dadurch gekennzeichnet sind, dass** sie induzierbar sind, um Tumornekrosefaktor-α, Interleukin-1β, Stickoxidsynthase-2, CX3CL1, CCL2, CXCL9 und/oder CXCL10 zu exprimieren.

5. Mikrogliale Vorläuferzellen für die Verwendung nach einem der vorherigen Ansprüche, wobei die mikroglialen Vorläuferzellen von induzierten pluripotenten Stammzellen (iPS-Zellen), embryonalen Stammzellen oder anderen pluri- oder multipotenten Stamm- oder Vorläuferzellen abstammen.

6. Mikrogliale Vorläuferzellen für die Verwendung nach einem der Ansprüche 1 und 3 bis 6, wobei es sich bei der malignen Neoplasie des Zentralnervensystems um eine maligne Neoplasie des Gehirns handelt.

7. Mikrogliale Vorläuferzellen für die Verwendung nach Anspruch 6, wobei es sich bei der malignen Neoplasie des Gehirns um ein Gliom handelt.

8. Mikrogliale Vorläuferzellen für die Verwendung nach Anspruch 7, wobei das Gliom aus Astrozytom und Oligodendrogliom ausgewählt ist.

## Revendications

1. Cellules précurseurs microgliales destinées à être utilisées dans le traitement d'un néoplasme malin du système nerveux central.

2. Cellules précurseurs microgliales destinées être utilisées selon la revendication 1, dans lesquelles les cellules précurseurs microgliales sont mélangées ou associées dans un récipient distinct à des agents chimio-attractifs pour les cellules immunitaires cytotoxiques et/ou à des substances immunostimulantes pour les précurseurs microgliaux.

3. Cellules précurseurs microgliales destinées être utilisées selon la revendication 1 ou 2, dans lesquelles les cellules précurseurs microgliales sont **caractérisées par** l'expression de CD45, CD11b, CD11c, CD14, CD16, CD36, CD68, l'intégrine alpha 4, l'intégrine bêta 1, CX3CR1, TREM2 et DAP12.

4. Cellules précurseurs microgliales destinées être utilisées selon la revendication 1 ou 3, dans lesquelles les cellules précurseurs microgliales sont **caractérisées en ce qu'**elles sont inductibles pour exprimer le facteur onconécrosant α, l'interleukine 1β, l'oxyde nitrique synthase 2, CX3CL1, CCL2, CXCL9 et/ou CXCL10.

5. Cellules précurseurs microgliales destinées être utilisées selon l'une quelconque des revendications précédentes, dans lesquelles les cellules précurseurs microgliales sont dérivées de cellules souches pluripotentes induites (iPS), de cellules souches embryonnaires ou autres cellules précurseurs ou souches pluripotentes ou multipotentes.

6. Cellules précurseurs microgliales destinées être utilisées selon l'une quelconque des revendications 1 et 3 à 6, dans lesquelles le néoplasme malin du système nerveux central est un néoplasme malin du cerveau.

7. Cellules précurseurs microgliales destinées être utilisées selon la revendication 6, dans lesquelles le néoplasme malin du cerveau est un gliome.

8. Cellules précurseurs microgliales destinées être utilisées selon la revendication 7, dans lesquelles le gliome est choisi parmi un astrocytome et un oligodendrogliome.
